Europäisches Patentamt

European Patent Office

Office européen des brevets

(19)

(11) **EP 0 854 846 B1**

(12) **EUROPÄISCHE PATENTSCHRIFT**

(45) Veröffentlichungstag und Bekanntmachung des Hinweises auf die Patenterteilung:
**17.04.2002 Patentblatt 2002/16**

(51) Int Cl.[7]: **C07D 217/20**, C07C 213/10, C07C 215/30, C07C 209/88

(21) Anmeldenummer: **96932524.0**

(22) Anmeldetag: **13.09.1996**

(86) Internationale Anmeldenummer:
**PCT/EP96/04030**

(87) Internationale Veröffentlichungsnummer:
**WO 97/11927 (03.04.1997 Gazette 1997/15)**

(54) **VERFAHREN ZUR RACEMATSPALTUNG**

METHOD OF RESOLVING RACEMIC MIXTURES

PROCEDE DE SEPARATION DE MELANGES RACEMIQUES

(84) Benannte Vertragsstaaten:
**AT BE CH DE FR GB IT LI NL SE**

(30) Priorität: **26.09.1995 DE 19535762**

(43) Veröffentlichungstag der Anmeldung:
**29.07.1998 Patentblatt 1998/31**

(73) Patentinhaber: **BASF AKTIENGESELLSCHAFT**
**67056 Ludwigshafen (DE)**

(72) Erfinder:
• **VIERGUTZ, Wolfgang**
**D-67067 Ludwigshafen (DE)**
• **KNOPFF, Jürgen**
**D-31675 Büggeburg (DE)**
• **BRASE, Walter**
**D-32469 Petershagen (DE)**

(56) Entgegenhaltungen:
**EP-A- 0 202 522**  **WO-A-92/00965**
**GB-A- 1 153 578**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein neues Verfahren zur Racematspaltung.

Es sind bereits eine Vielzahl von Racematspaltungen bekannt. Trotzdem gibt es bei Racematspaltungen immer wieder Probleme, weil die Spaltreagenzien nicht stabil genug sind, zu öligen und schlecht isolierbaren Antipoden führen, nur sehr umständlich anwendbar sind oder praktisch nicht mit den Racematen reagieren. Es besteht deshalb ein großes Interesse daran, für bestimmte Gruppen von Racematen geeignete Spaltreagenzien zu finden.

WO 92/00965 offenbart ein Verfahren, in dem Tetrahydropapaverin beispielsweise mit Hilfe von N-Acetylleucin in seine Antipoden gespalten wird. Diese Racematspaltung läuft nach einem 3-Stufen-Verfahren ab:

    a) Fällung des S-Enantiomeren mit N-Acetylleucin,

    b) Fällung des rohen (gewünschten) R-Enantiomeren mit N-Acetylleucin und

    c) Umkristallisation des an N-Acetylleucin gebundenen R-Enantiomeren.

Dieses Verfahren besitzt den entscheidenden Nachteil, daß mit dem N-Acetylleucin praktisch nur die natürlich vorkommende optisch aktive Spaltsäure zur Verfügung steht, die zuerst das unerwünschte diastereomere S-Salz des Tetrahydropapaverins ausfällt. Erst aus der mit dem R-Antipoden angereicherten Mutterlauge kann das Salz dieses Antipoden ausgefällt werden.

Es ist weiter bekannt, daß man mit (+)-2,4-Dichlorphenoxypropionsäure racemisches 2-Amino-1-butanol in seine Antipoden trennen kann (EP-A 202 522).

Es wurde nun ein einfacheres Verfahren zur Racematspaltung gefunden.

Gegenstand der Erfindung ist ein Verfahren zur Racemattrennung von Ephedrin oder Tetrahydropaverin in an sich bekannter Weise, dadurch gekennzeichnet, daß man die Spaltung mit den optischen Antipoden von (+)- bzw. (-)-2-(2,4-Dichlorphenoxy)-propionsäure vornimmt.

Die Racematspaltung läßt sich bei Temperaturen zwischen 0°C und dem Siedepunkt des verwendeten Lösungsmittels durchführen. Am einfachsten ist das Arbeiten bei Raumtemperatur.

Die Spaltung läßt sich in üblichen Lösungsmitteln, wie niederen Alkoholen, Aceton, Toluol, Xylolen, Ethern, Tetrahydrofuran und Essigsäureethylester, durchführen. Normalerweise verwendet man gesättigte Lösungen. Vorzugsweise wird Isopropanol oder Toluol verwendet.

Aus dem bei der Racematspaltung entstehenden Salz läßt sich in wäßriger Lösung bei pH 7,5 - 10 die Base freisetzen und mit einem in Wasser nicht löslichen Lösungsmittel wie Ether, Toluol oder einem Xylol extrahieren.

Das neue Verfahren zeichnet sich dadurch aus, daß die Spaltreagenzien leicht erhältlich und stabil sind. Weiter besitzen sie eine hohe Selektivität und liefern daher die Antipoden in guten Ausbeuten und in sehr hoher Reinheit.

Gegenstand der Erfindung sind weiter die Salze des Ephedrins und Tetrahydropapaverins speziell das L-Ephedrin-D-dichlorphenoxypropionat und insbesondere das (+)-THP-D-24-dichlorphenoxypropionat.

Beispiel 1

1,029 g (3 mmol) THP (THP = Tetrahydropapaverin = 1-[(3,4-Dimethoxyphenyl)methyl]-6,7-dimethoxy-1,2,3,4-tetrahydroisochinolin) wurde in 2 ml Isopropanol aufgenommen, mit einer heißen Lösung von 0,707 g (3 mmol) D-2,4-Dichlorphenoxypropionsäure in 8 ml Isopropanol versetzt und kurz zum Rückfluß erhitzt. Die entstandenen Kristalle wurden nach dem Abkühlen auf Raumtemperatur abgesaugt, mit Isopropanol gewaschen und getrocknet. Es blieben 0,9 g eines Produkts zurück, welches überwiegend aus (+)-THP-D-2,4-dichlorphenoxypropionat bestand.

Das erhaltene Produkt wurde in 5 ml Wasser gelöst. Nach Einstellen des pH-Wertes der Lösung auf pH 8 wurde mit Toluol extrahiert. Es wurden nach Entfernen des Toluols 0,48 g (93,3 %) des (+)-Enantiomeren in einer optischen Reinheit von 95,6 % erhalten.

Beispiel 2

17,15 g (50 mmol) THP wurden mit 5,85 g (25 mmol) D-2,4-Dichlorphenoxypropionsäure in 80 ml Isopropanol unter Rühren erhitzt. Bei 45°C lag eine klare Lösung vor. Bei 50°C wurde die Lösung mit dem (+)-Isomeren Salz angeimpft. Anschließend wurde im Verlauf von 2 h unter Rühren auf Raumtemperatur abgekühlt. Zur Verdünnung wurden noch 40 ml Isopropanol zugegeben. Die ausgefallenen Kristalle wurden abgesaugt, mit 30 ml Isopropanol gewaschen und getrocknet. Aus dem so erhaltenen Salz wurde das THP analog Beispiel 1 freigesetzt.

Man erhielt 5,2 g (61 %) (+)-THP in einer optischen Reinheit von 90,1 %.

Beispiel 3

1,715 g (5 mmol) THP und 0,588 g (2,5 mmol) D-2,4-Dichlorphenoxypropionsäure wurden in 10 ml Toluol bei der Rückflußtemperatur gelöst und anschließend langsam abgekühlt. Die entstandenen Kristalle wurden abgesaugt und getrocknet. Die Isolierung des (+)-THP erfolgte analog Beispiel 1. Es wurden 0,85 g (98,8 %) (+)-THP erhalten. Die optische Reinheit betrug 96,1 %.

Beispiel 4

Eine Lösung von 17,15 g (50 mmol) THP in 55 ml Toluol wurde bei 60°C mit einer Lösung von 5,88 g (25 mmol) D-2,4-Dichlorphenoxypropionsäure in 55 ml Toluol bei 60°C gerührt und zur Kristallisation gebracht. Nach Absaugen und Auswaschen der Kristalle mit Toluol wurde getrocknet.

Die Kristalle wurden analog Beispiel 1 zum (+)-THP aufgearbeitet. Die Ausbeute betrug 8,6 g (98,3 %), die optische Reinheit des (+) -THP lag bei 98,7 %.

Beispiel 5

8,25 g (50 mmol) D,L-Ephedrin und 5,88 g (25 mmol) D-2,4-Dichlorphenoxypropionsäure wurden in 100 ml Toluol aufgenommen. Nach dem Erwärmen bis zum Rückfluß wurde langsam abgekühlt, die Kristalle wurden abgesaugt und mit Toluol gewaschen. Nach dem Trocknen wurden 4,2 g (42 %) Ephedrin-Salz erhalten. Nach Freisetzen der Base analog Beispiel 1 und deren Überführung in das Hydrochlorid erhielt man L-Ephedrin-Hydrochlorid in einer Gesamtausbeute von 40 %, $[\alpha]_D^{20}$ : - 33, 70.

**Patentansprüche**

1. Verfahren zur Racemattrennung von Ephedrin oder Tetrahydropapaverin in an sich bekannter Weise, **dadurch gekennzeichnet, daß** man die Spaltung mit (+)- bzw. (-)-(2)-2-(2,4-Dichlorphenoxy)-propionsäure vornimmt.

2. (+)-THP-D-2,4-dichlorphenoxypropionat.

3. L-Ephedrin-D-2,4-dichlorphenoxypropionat.

**Claims**

1. A process for resolving racemates of compounds of ephedrine or tetrahydropapaverine in a manner known per se, wherein the resolution is carried out with (+)- or (-)-(2)-2-(2,4-dichlorophenoxy)propionic acid .

2. (+)-THP D-2,4-dichlorophenoxypropionate.

3. L-Ephedrine D-2,4-dichlorophenoxypropionate.

**Revendications**

1. Procédé pour la résolution des racémates de l'éphédrine ou de la tétrahydropapavérine de manière connue en soi, **caractérisé par le fait que** l'on procède à la résolution avec respectivement l'acide (+)- ou l'acide (-)-(2)-2-(2,4-dichlorophénoxy)propionique.

2. Le D-2,4-dichlorophénoxypropionate de (+)-THP.

3. Le D-2,4-dichlorophénoxypropionate de L-éphédrine.